# EUROPEAN PATENT SPECIFICATION

(11) **EP 2 901 867 B1**
(45) Date of publication and mention of the grant of the patent: **18.10.2017**
(21) Application number: 14197402.2
(22) Date of filing: 11.12.2014
(51) Int. Cl.: A23L 19/20, A23B 7/155, C12R 1/01

(54) **Method for production of fermented table olives**
Verfahren zur Herstellung fermentierter Tafeloliven
Procédé pour produire des olives de table fermentées

(30) Priority: 11.12.2013 IT MI20132063
(43) Date of publication of application: 05.08.2015
(73) Proprietor: CONSIGLIO NAZIONALE DELLE RICERCHE, 00198 Roma (IT)
(72) Inventor: Bleve, Gianluca, 73100 Lecce (IT); Tufariello, Maria, 70032 Bitonto (IT); Durante, Miriana, 73045 Leverano (IT); Perbellini, Ezio, 73022 Corigliano D'Otranto (IT); Mita, Giovanni, 73025 Martano (IT); Ramires, Francesca Anna, 73047 Monteroni di Lecce (IT); Grieco, Franceso, 73100 Lecce (IT); Logrieco, Antonio Franceso, 70126 Bari (IT)
(74) Representative: Zaccaro, Elisabetta

(56) References cited:
- ANTONIO BEVILACQUA ET AL: "Selection of Yeasts as Starter Cultures for Table Olives", JOURNAL OF FOOD SCIENCE, vol. 78, no. 5, 9 April 2013 (2013-04-09), pages M742-M751, XP055132231, ISSN: 0022-1147, DOI: 10.1111/1750-3841.12117
- DATABASE FSTA [Online] INTERNATIONAL FOOD INFORMATION SERVICE (IFIS), FRANkFURT-MAIN, DE; 1996, PAPOFF C M ET AL: "Influence of some biotechnological combinations on the sensory quality of Manna green table olives.", XP002727974, Database accession no. FS-1996-09-J-0096
- DATABASE FSTA [Online] INTERNATIONAL FOOD INFORMATION SERVICE (IFIS), FRANkFURT-MAIN, DE; 1977, VAUGHN R H ET AL: "Acid-forming yeasts and fermentation of olives.", XP002727975, Database accession no. FS-1977-07-J-0992
- DATABASE FSTA [Online] INTERNATIONAL FOOD INFORMATION SERVICE (IFIS), FRANkFURT-MAIN, DE; 2009, SILVESTRI V ET AL: "Technological ability of lactic acid bacteria starter for table olives production.", XP002727976, Database accession no. FS-2009-12-Jg5589

## Description

### Field of the invention

The invention relates to a process for the fermentation of table olives which has the advantage of allowing to standardize and reduce the time for the whole fermentation process, and to evaluate the process independently from subjective and empirical criteria currently used by manufacturers, thereby increasing quality and safety levels of the final product, compared to traditional methods. Moreover, starter cultures and their use in fermentation of two cultivars of table olives are also described.

### State of the art

Olive production is a major agricultural practice in Mediterranean countries such as Italy, Greece and Spain. Table olives are one of the most important traditional fermented vegetable products of Southern European nations and their production and consumption are constantly increasing. Olive production worldwide consists of 2 million tons per year, 38% of which is produced in Spain, Italy and Greece. Italian production of table olives is around 70000 tons, and accounts for about 10% of world production and 20% of the production in the European Union. Natural Black olives, produced by the Greek method, are one of the most important products on the international market, along with green olives produced by the Spanish method and oxidized black olives, well known as olives produced by the Californian method.

According to the Greek method, olives are immersed in a brine solution containing a salt concentration comprised between 6-10% (weight/volume). Under these conditions, natural fermentation, which requires 8-12 months to be completed, takes place and is operated by an epiphytic microbial consortium consisting of yeasts and lactic acid bacteria (LAB).

With current technology, lactic fermentation by lactic acid bacteria is considered the most important step in the process of production of table olives, because it is responsible for: i) debittering of olives; ii) decrease of brine pH, with a reduced risk for possible growth of spoiling or pathogenic microorganisms, ii) formation of organoleptic characteristics and of an optimal consistency of the finished product.

A possible role of yeasts in the preparation and storage of table olives has been recently also considered. In fact, such microorganisms can positively influence fermentation through their production of volatile molecules and metabolites capable of improving the organoleptic characteristics of the final product, through the release of nutrients favouring growth of lactic acid bacteria (LAB) and due to their ability to operate bio-reduction of undesired phenolic compounds. However, yeasts may also cause problems during the production process, as they can be responsible for formation of gas pockets, olive softening, package swelling, brine opacity or production of undesirable flavors and odors.

The yeasts associated with table olives belong to genera such as *Aureobasidium, Candida, Cryptococcus, Issatchenkia, Kluyveromyces, Pichia, Rhodotorula, Saccharomyces, Wickerhamomyces* and Zygotorulaspora, and in particular the species *S. cerevisiae, VV. anomalus, P. membranifaciens, P. galeiformis, C. boidinii, C. diddensiae* and *K. lactis.*

At present, the control of the process and the production of fermented olives depend solely on empirical experience of the producers who, according to subjective criteria, assess when olives are ready for consumption and establish the levels of quality and safety of the final product. Current fermentation processes are spontaneous, unpredictable and strongly influenced by physical-chemical conditions, availability of fermentable substrates and salt content, as well as by microorganisms present as drupe contaminants.

Bevilacqua A., et al (J Food Sci. 2013 May;78(5):M742-51) relates to table olive fermentation, and describes the selection of yeast strains, isolated from the Bella di Cerignola table olives, in relation to different enzymatic traits and to the capacity to grow in different conditions. In particular, yeast selection is described on the basis of the capacity of the strain to grow at 15°C and with high concentration of NaCl, as well as at alkaline pHs and possessing an interesting β-glucosidase activity. The isolated are *Kluyveromyces lactis, Wickerhamomyces anomalus* and *Candida norvegica.*

Different yeast and bacterial strains have been described:
- FSTA database entry FS-1996-09-J-0096 regards microbial brine inocula for improving the sensory quality of green table olives;
- FSTA database entry FS-1977-07-J-0992 regards the detection of yeast strains in several brines where green olives were fermented. Different yeasts were studied for their ability to grow at the different percentages of NaCl, used in brine soaking;
- FSTA database entry FS-2009-12-Jg5589 regards the selection of lactic acid bacteria.

For many years, the search for starters to be applied to fermentation of table olives has been focused on the activity of LAB. Based on this research activity, the use of starter cultures of lactic acid bacteria, including *Lactobacillus plantarum, L. pentosus* in single culture or mixture has been proposed to control fermentation and produce high quality products. The importance and potential applications of yeasts in the process of production of table olives have begun to be considered only in recent years.

The particular difficulty in determining performance and completion of the fermentation process, together with the current lack of chemico-physical or microbiological controls to determine when olives are ready to be marketed and consumed, are the factors behind the substantial absence of positive approaches to determine the optimal timing to achieve the finished product.

The present invention allows to follow the entire fermentation process and proposes specific chemical parameters to determine, respectively, yeast and LAB activity to be used as indicators and descriptors of good or bad fermentation performance and to determine unequivocally the completion of the whole fermentation process.

Therefore, the aim of the present invention is to allow to perform the fermentation process for production of fermented olives in a controlled manner and with reduced timing independently from subjective criteria empirically established by an individual producer.

### Summary of the invention

The above stated purpose has been achieved through development of a method for the production of fermented table olives wherein the olive sample is sequentially inoculated with a yeast strain and, after an appropriate time, with a lactic acid bacterium.

The inventors have in fact identified for the first time a process for fermentation of table olives involving the sequential use of yeast and subsequently of lactic acid bacteria.

Therefore, the invention relates to a method for production of fermented table olives comprising the steps of:
a. soaking the olives in brine;
b. inoculating the product obtained from step a. with a yeast culture;
c. incubating the product obtained from step b. to enable alcoholic fermentation;
d. inoculating the fermented product obtained from step c. with a Lactic Acid Bacterium culture;
e. incubating the product obtained from step d. to enable lactic fermentation.

Under yet another aspect, in a preferred form of realization, the invention relates to a starter culture comprising at least one yeast of the strain *Saccharomyces cerevisiae* (cod. DSMZ 27800) and at least one lactic acid bacterium of the strain *Lactobacillus plantarum* (cod. DSMZ 27925), and its use for fermentation of an olive sample of the Italian cultivar Leccino.

### Description of the figures

The invention will now be described in detail and in reference to the attached Figures, wherein:

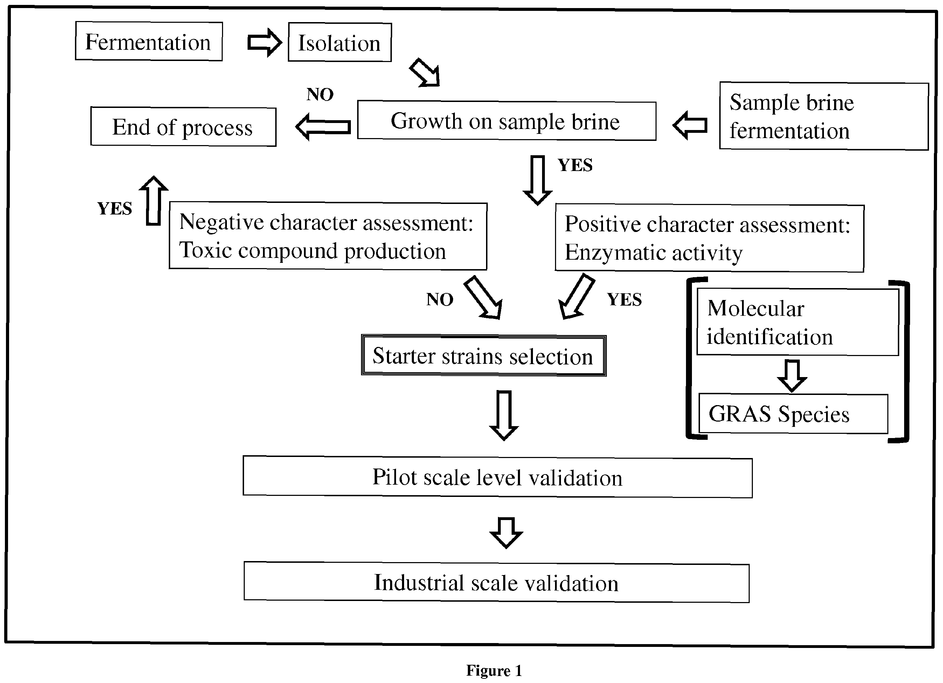

- Figure 1 shows the flow chart of the steps to be carried out in order to select yeast/LAB starter cultures used as indigenous starter.

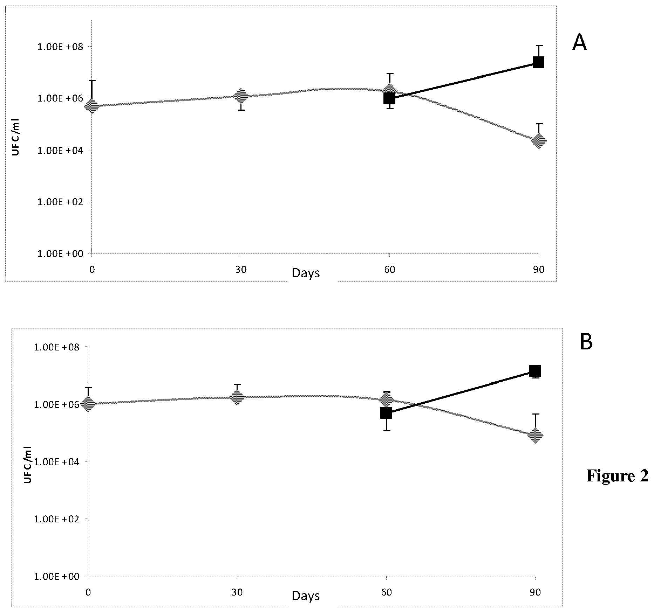

- Figure 2 shows the growth profile of (A) Yeast (DSMZ 27800) and LAB (DSMZ 27925) starter cultures inoculated to ferment Leccino cultivar olives and (B) Yeast (DSMZ 27801) and LAB (DSMZ 27926) starter cultures inoculated to ferment Kalamata cultivar olives.

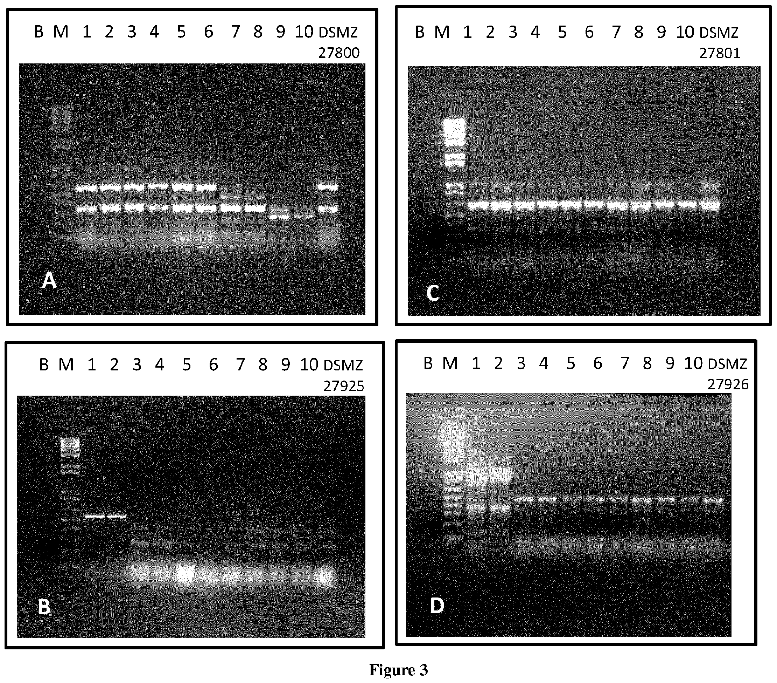

- Figure 3 shows the analysis of dominance of (A) Yeast (DSMZ 27800) and (B) LAB (DSMZ 27925) starter cultures at the end of fermentation of Leccino cultivar olives and (C) Yeast (DSMZ 27801) and (D) LAB (DSMZ 27926) starter cultures at the end of fermentation of Kalamata cultivar olives. Line 1: Blank sample, Line 2: DNA Marker, Line 3-12: RAPD profiles of 10 different microbial isolates, Line 13: profile of inoculated yeast or LAB starter.

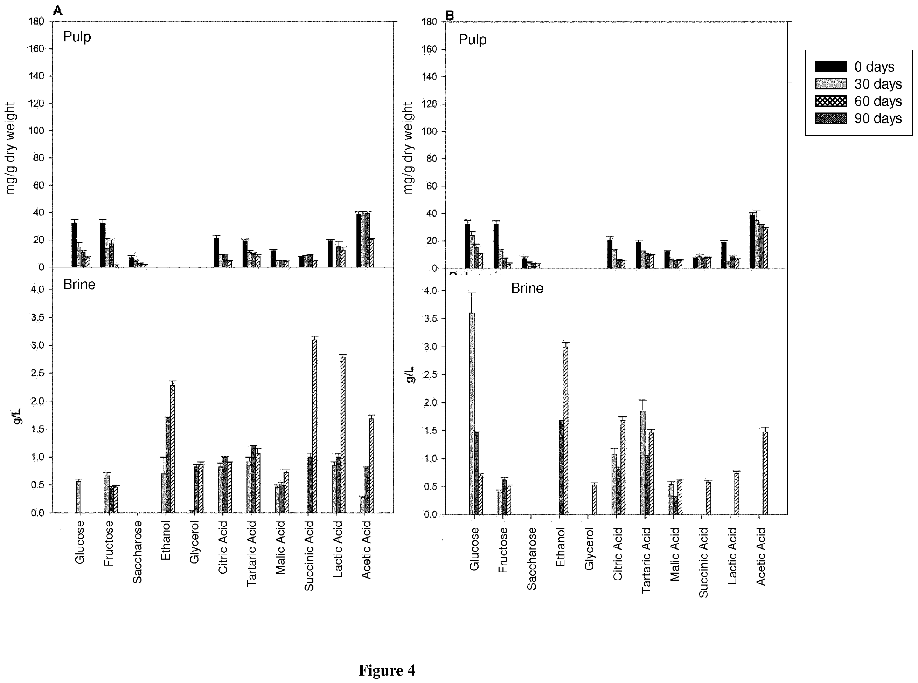

- Figure 4 shows variations in the concentration of sugars, organic acids and alcohols in the pulp and in the brine of Leccino olives (A) fermented by yeast (DSMZ 27800) and LAB (DSMZ 27925) starters and (B) fermented by spontaneous fermentation.

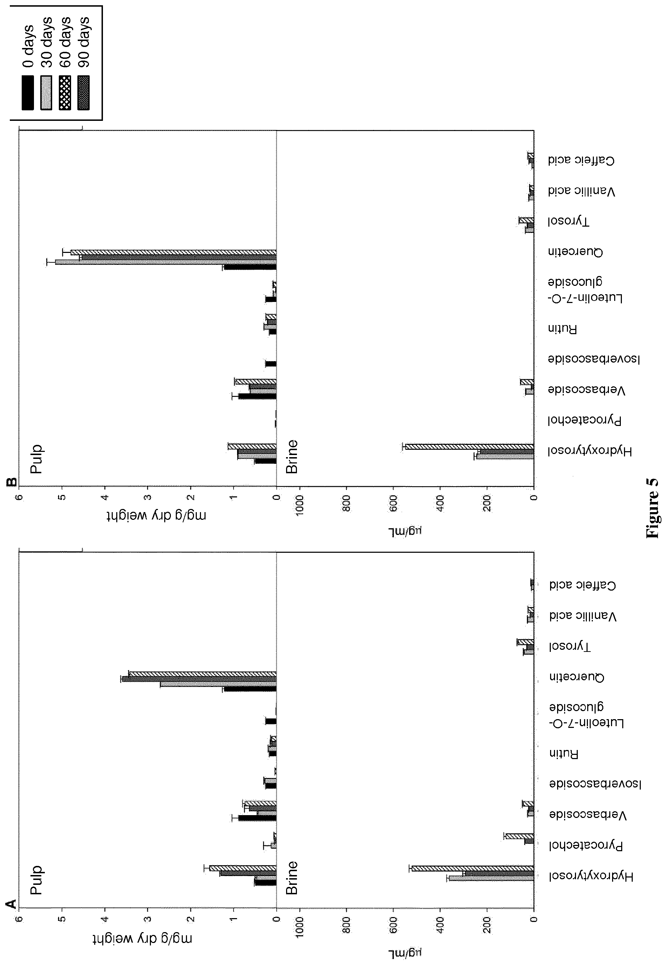

- Figure 5 shows variations in the concentration of sugars, organic acids and alcohols in the pulp and in the brine of Kalamata olives (A) fermented by yeast (DSMZ 27801) and LAB (DSMZ 27926) starters and (B) fermented by spontaneous fermentation.

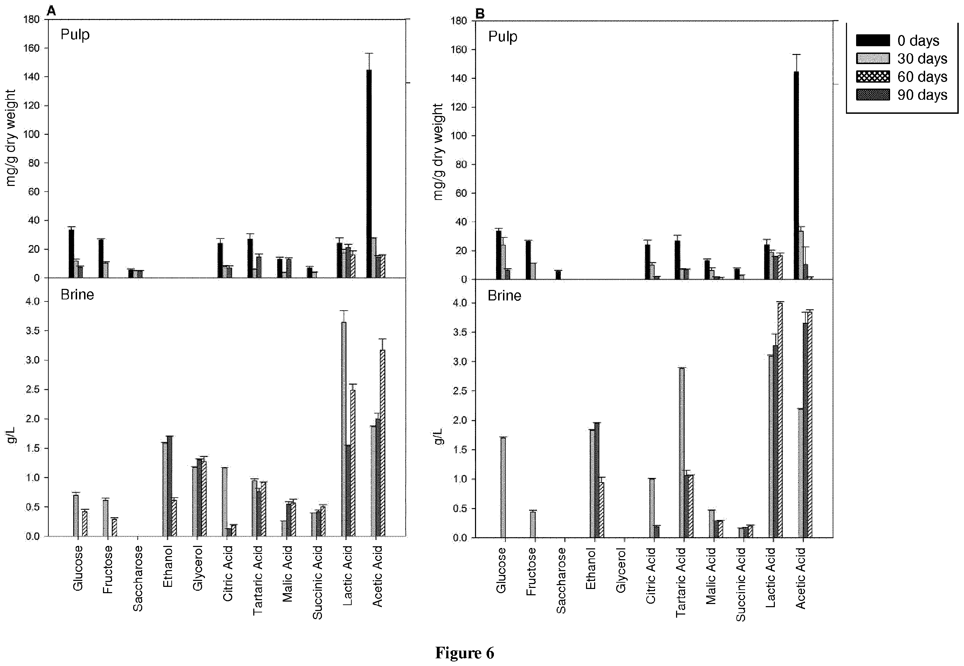

- Figure 6 describes variations in the concentration of monophenols and polyphenols present in the pulp and in the brine of Leccino olives (A) fermented by yeast (DSMZ 27800) and LAB (DSMZ 27925) starters and (B) fermented by spontaneous fermentation.

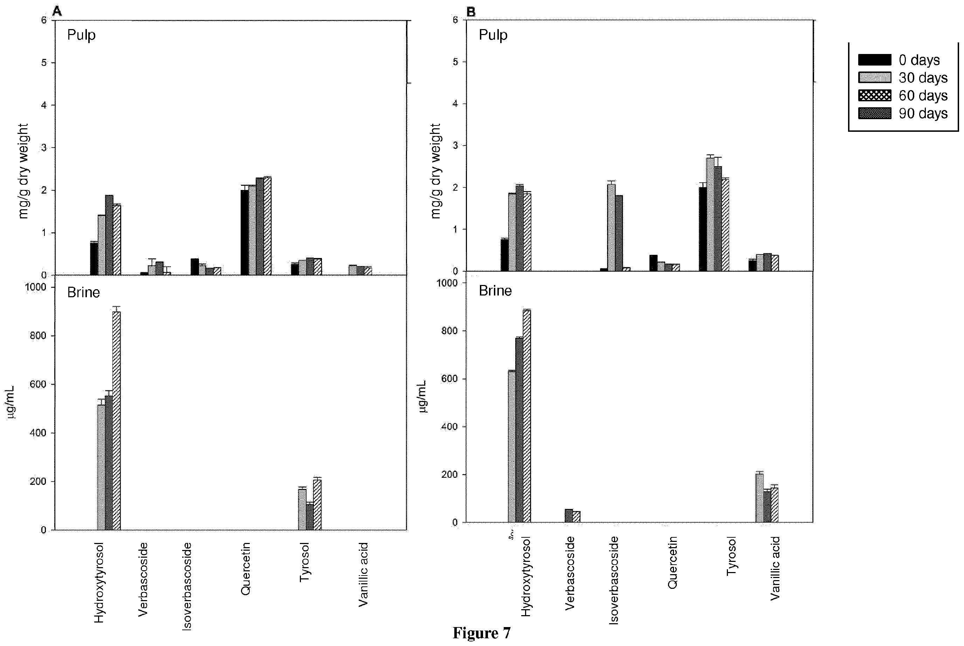

- Figure 7 shows variations in the concentration of monophenols and polyphenols present in the pulp and in the brine of Kalamata olives (A) fermented by yeast (DSMZ 27801) and LAB (DSMZ 27926) starters and (B) fermented by spontaneous fermentation.

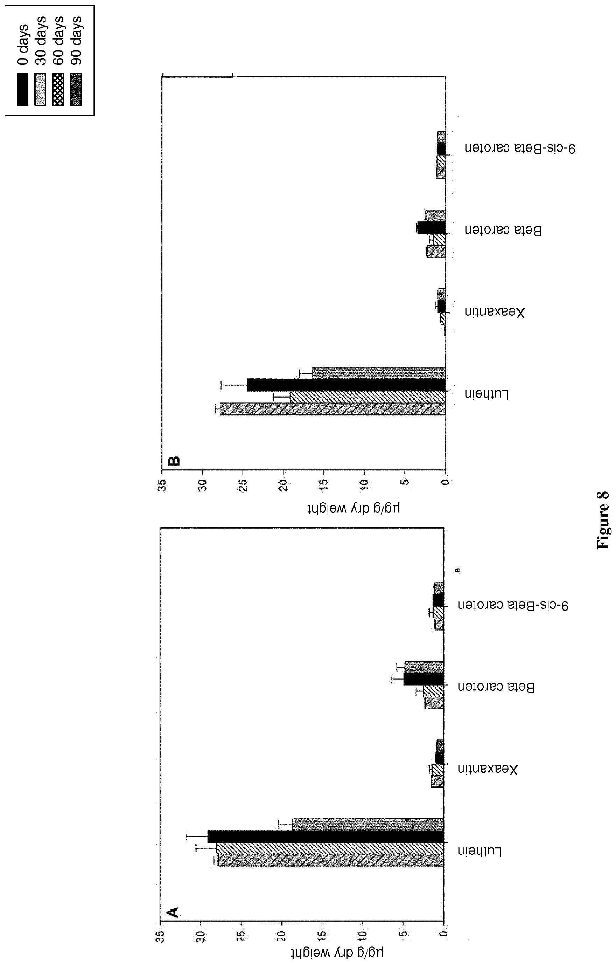

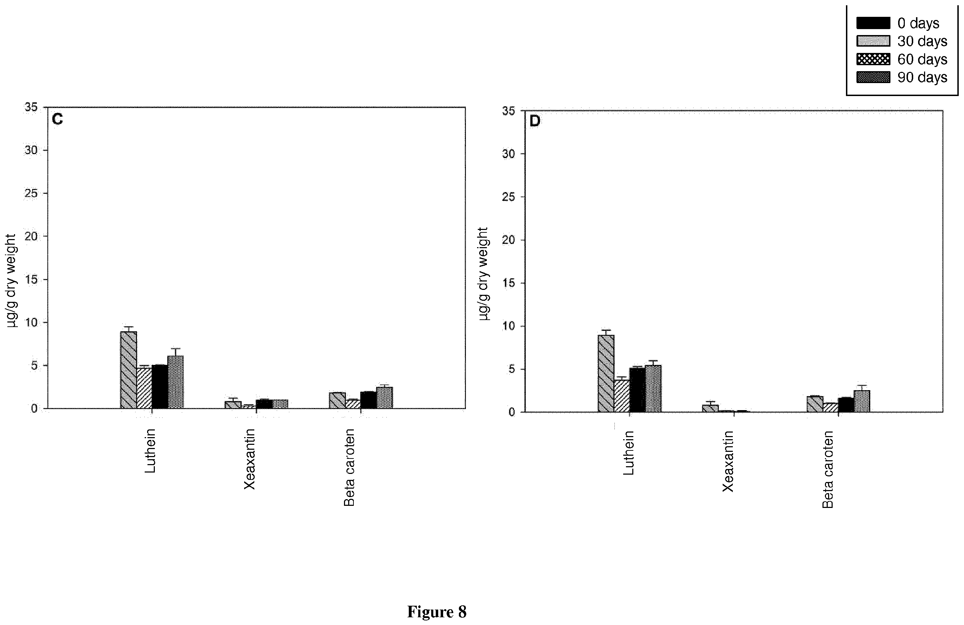

- Figure 8 shows variations in carotenoids concentration in the pulp of (A) Leccino olives fermented by yeast (DSMZ 27800) and LAB (DSMZ 27925) starter, (B) Leccino olives fermented by spontaneous fermentation, (C) Kalamata olives fermented by yeast (DSMZ 27801) and LAB (DSMZ 27926) starters and (D) Kalamata olives fermented by spontaneous fermentation.

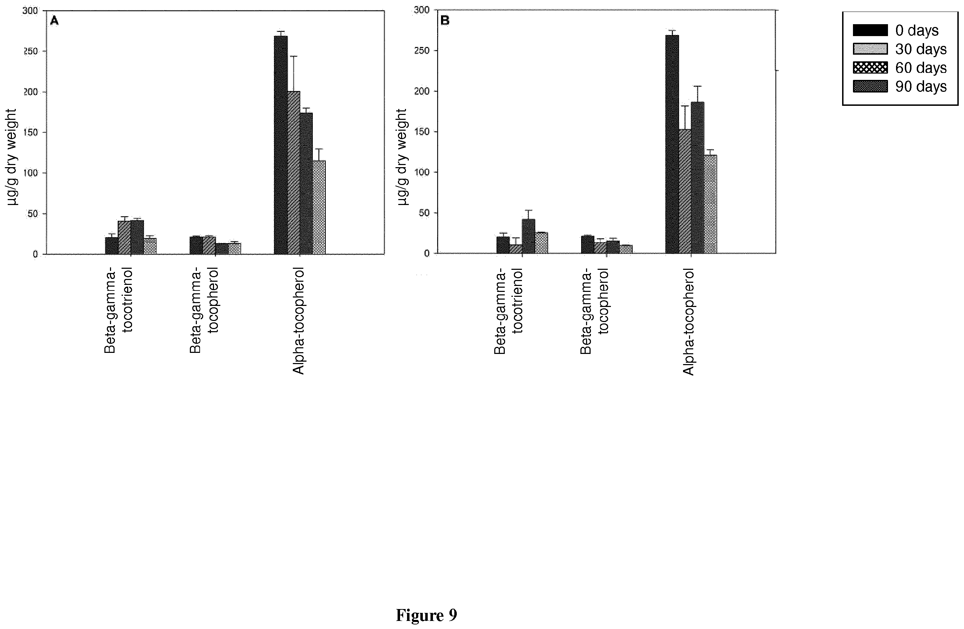

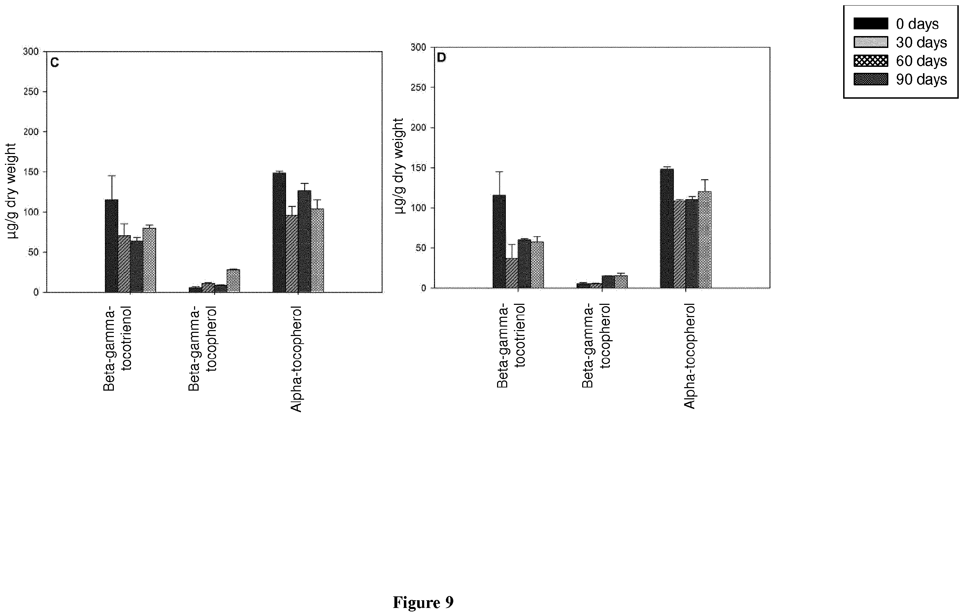

- Figure 9 shows variations in vitamin E concentration in the pulp of (A) Leccino olives fermented by yeast (DSMZ 27800) and LAB (DSMZ 27925) starters, (B) Leccino olives fermented by spontaneous fermentation, (C) Kalamata olives fermented by yeast (DSMZ 27801) and LAB (DSMZ 27926) starters and (D) Kalamata olives fermented by spontaneous fermentation.

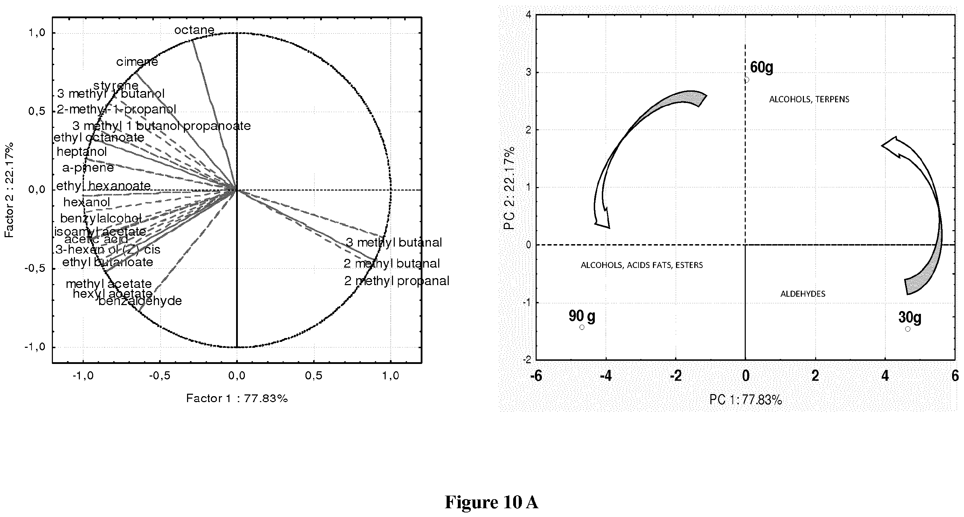

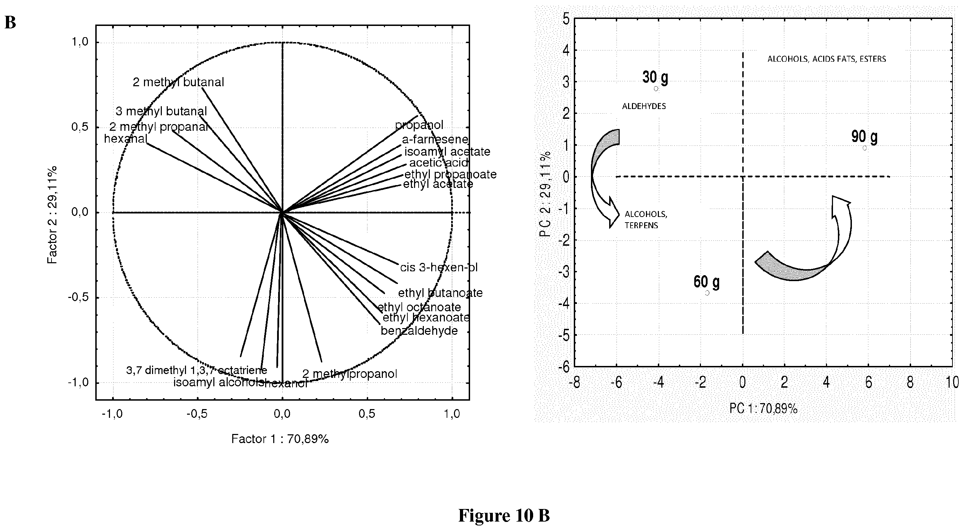

- Figure 10. Principal components analysis (PCA) of volatile compounds associated with fermented table olives of the variety (A) Leccino and (B) Kalamata. PCA analysis was conducted using as variables the data obtained from the analysis of concentrations and presence of volatile compounds during three different fermentation times. The figure is a bi-plot, showing values for the samples and variables in PC1-PC2 planar format. The figure also shows the major classes of molecules representative of the first part of fermentation (30 g), fermentation by yeast (60 g) and fermentation by LAB (90 g).

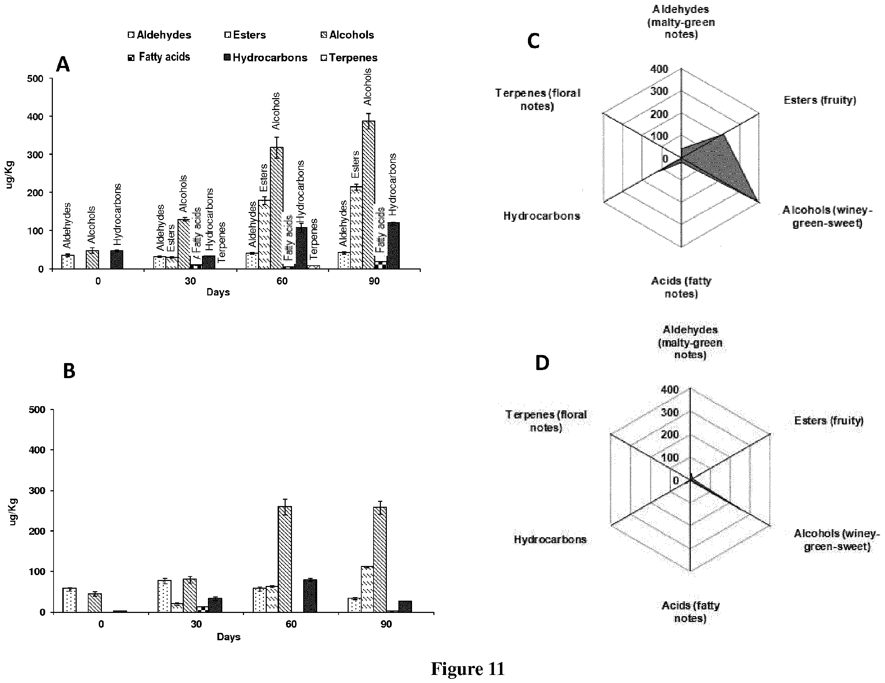

- Figure 11 shows variations in the concentration of the major classes of volatile compounds associated with Leccino olives fermented (A) by sequential inoculation of yeast (DSMZ 27800) and LAB (DSMZ 27925) starter cultures (B) by natural fermentation. Radar plot representation of sensorial attributes associated with the classes of volatile molecules identified in olives fermented by (C) sequential inoculation of yeast (DSMZ 27800) and LAB (DSMZ 27925) starter cultures (D) natural fermentation.

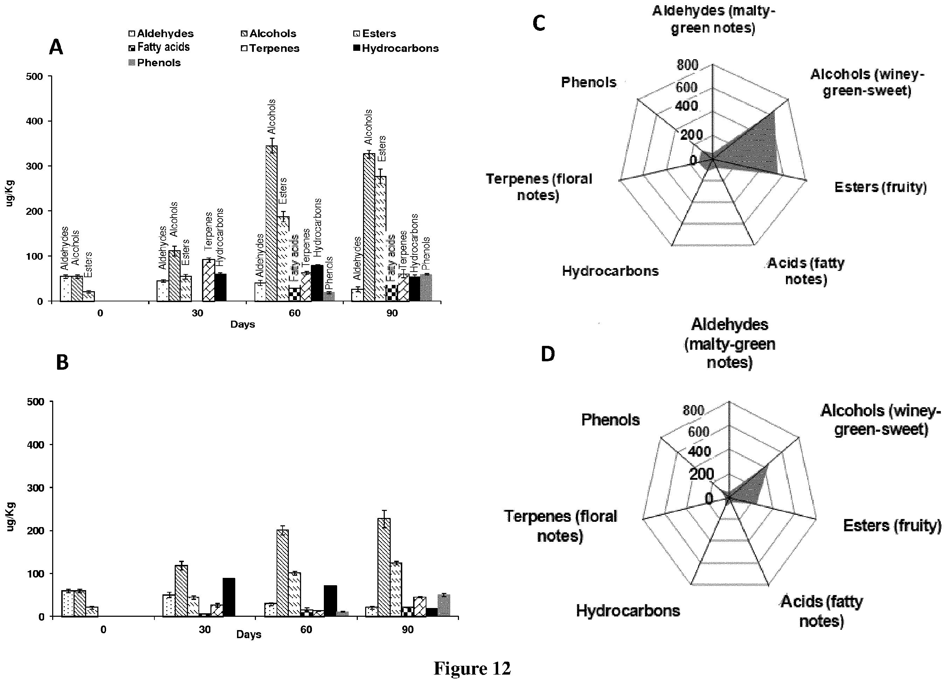

- Figure 12 describes variations in the concentration of the major classes of volatile compounds associated with Kalamata olives fermented by (A) sequential inoculation of yeast (DSMZ 27801) and LAB (DSMZ 27926) starter cultures (B) natural fermentation. Radar plot representation of sensorial attributes associated with the classes of volatile molecules identified in olives fermented by (C) sequential inoculation of yeast (DSMZ 27801) and LAB (DSMZ 27926) starter cultures (D) natural fermentation.

### Detailed description of the invention

The invention therefore relates to a method for production of fermented table olives wherein the sample of olives is sequentially inoculated with a first yeast starter culture followed by a culture of the lactic acid bacterium.

Currently, the production of fermented olives depends on the experience of producers who determine, according to subjective and empirical criteria, when olives are ready to be consumed and the quality and safety levels of the final product. At present fermentation processes are spontaneous, unpredictable and strongly influenced by physical-chemical conditions, availability of fermentable substrates and salt content, as well as by microorganisms present as drupe contaminants.

According to a specific protocol, the inventors have selected isolates of yeasts and lactic acid bacteria, and have developed through their use a manufacturing process capable of: i) increasing organoleptic and nutritional properties of fermented table olives, ii) driving a controlled fermentation process, iii) reducing the time required to obtain the finished product.

Therefore, the invention relates to a method for production of fermented table olives comprising the steps of:
a. soaking the olives in brine;
b. inoculating the product obtained from step a. with a yeast;
c. incubating the product obtained from step b. in order to allow alcoholic fermentation;
d. inoculating the fermented product obtained from step c. with a lactic acid bacterium;
e. incubating the product obtained from step d. to allow lactic fermentation.

Brine is prepared by dissolving sodium chloride in the appropriate amount of water to obtain a percentage comprised between 6 and 12% (weight/volume).

The experimental results obtained have shown that the method of the invention allows to reduce the fermentation time from 6-12 months, usually required to complete the empirical production process, to a maximum of three months.

The above method also provides the advantage of controlled fermentation processes for production of fermented olives independently from subjective and empirical criteria of producers. By monitoring fermentation-associated chemical parameters serving as indicators of fermentation processes, it allows to follow the progress of such processes and promptly intervene with the necessary corrections. In a preferred form, the method for production of fermented table olives according to the present invention comprises the steps of:
a. soaking the olives in brine.
   In phase a., the olive:brine ratio varies from about 3:1, for olives with a larger calibre (from 60/70 pieces per Kg up to 210 pieces per Kg) to about 2:1 for olives with a smaller calibre (from 220 pieces per Kg up to 410 pieces per kg), as established by the COI/OT/NC no.1 December 2004;
b. inoculating the product obtained from step a. with a yeast, using a ratio 1:300 between the volume of the yeast inoculum and the volume of brine to be inoculated in phase b;
c. incubating the product obtained from step b. to enable alcoholic fermentation at a variable temperature, from about 6°C to 30°C, for a variable time from about 45 to about 60 days;
d. inoculating the fermented product obtained from step c. with a lactic acid bacterium, using a ratio of about 1/300 between LAB starter and fermented product, i.e. brine volume;
e. incubating the product obtained from step d. to enable lactic fermentation at a temperature ranging from about 6°C to 30°C for a time ranging from about 15 to about 30 days.

The present invention can be applied under ambient conditions in a wide temperature range corresponding to the normal temperatures in the Mediterranean Basin during the months of olive processing (October to May). This possibility makes the present invention exploitable under normal working conditions for Italian and Greek companies that do not use systems for temperature control of the containers where fermentation of table olives takes place, either barrels or silos, and carry out the entire process at the ambient temperature.

The inoculation is made in a sequential mode: it is initiated with the inoculation of the yeast inoculum capable of producing the first fermentation, followed by inoculation of the lactic acid bacterium responsible for lactic fermentation. This procedure allows to mimic the natural spontaneous fermentation process. In this way, yeasts are capable of performing the first fermentation and, through the release of nutrients and the reduction of antibacterial polyphenolic compounds, determine the ideal conditions for lactic acid bacteria to carry out alcohol-lactic fermentation at best.

Preferably the method according to the present invention is a method allowing the production of fermented table olives, wherein the example of said olives are chosen from the group consisting of Italian olives of Leccino cultivar and Greek olives of Kalamata cultivar.

Leccino is one of the olive cultivars most widely spread in the Italian territory. The Leccino cultivar originates from central and southern Italy (Tuscany, Lazio, Abruzzo, Molise and Puglia) and is currently the most cultivated worldwide and widespread in Argentina, Jordan, Israel, South Africa, California, New Zealand and Australia. Drupes have an average size (2-2.5 g), ellipsoidal shape and are slightly asymmetrical with rounded apex and flattened base. The cultivar is characterized by an early and simultaneous fruit ripening, and the oil produced from this cultivar is remarkably widespread. At the harvesting, olives are black-violet and oil yield varies between 17-22%. This cultivar is resistant to low temperatures, temperature changes, winds, fog, olive knot, dry rot, olive leaf spot. This cultivar has found use for production of table olives of shimmering or black type. It is traditionally produced by the Greek method involving natural fermentation in brine from 8-12% sodium chloride (weight/volume), reaching complete debittering after about 8-10 months. The method described herein, which employs sequentially the selected starter cultures of yeast and lactic acid bacteria, allows to reach complete debittering within maximum 3 months and to enrich the volatile and sensorial profile of olives with notes of fruity, floral, winey, herbaceous, malty, and reduces volatile hydrocarbons and phenols (Fig. 11 and 12) while ensuring the maintenance of the nutritional characteristics represented by phenolic and isoprenoid-type components (Figures 6-9).

The Greek olive Kalamata originates from Messenia region. This only black olive is produced in southern Peloponnese and in the Agrinion area. It has a high weight and asymmetrical elongated shape with sharp apex and base. In normal seasons, the harvesting of black olives begins in October and ends in February, the color is given only by longer exposure to the sun.

According to the Greek method, olives are immersed in brine solution containing a salt concentration comprised between 6-10% (weight/volume). They only require fermentation in water and salt for a long period - indeed they are ready for consumption after about 4-6 months from the harvesting - and can be cut vertically in order to reduce the fermentation time. Residual levels of polyphenols in olives after processing are responsible for the slightly bitter taste of the finished product. The preparation of Kalamata olives also requires the addition of olive oil and vinegar. Fermentation is carried out by mixed populations of microorganisms consisting of epiphytic microflora of yeasts and lactic acid bacteria. Currently, the fermentation process relies on the ability of epiphytic microflora to self-select and accomplish this process efficiently, without any possibility to predict performance, quality and healthiness of the finished product that will be obtained. Uncontrollable abnormal fermentations often compromise the final product with respect to quality and safety for the consumer. To date, there are no systems to monitor the development of the process from the point of view of quality and safety, and are not available parameters that allow to obtain a product with recognizable organoleptic characteristics over the years.

The method described in the present invention has the advantage of allowing for the production of fermented table olives, wherein in case of Italian olives of Leccino cultivar, the yeast used to supplement the olive sample is a selected *Saccharomyces cerevisiae* yeast strain (cod. DSMZ 27800) and the strain of lactic acid bacteria used to inoculate the olive sample partially fermented by yeast (first fermented product) is a selected strain of *Lactobacillus plantarum* (cod. DSMZ 27925).

The yeast *Saccharomyces cerevisiae* strain for fermentation of olives of the Italian Leccino cultivar was filed on October 22, 2013 at the Leibniz-Institut DSMZ-Deutsche Sammlung von Mikroorganismen und Zellkulturen GmbH, with the deposit number Saccharomyces cerevisiae Leccino-Y1 (LI-180 -7) (cod. DSMZ 27800). The *Lactobacillus plantarum* bacterial strain for fermentation of olives of the Italian cultivar Leccino was filed on October 22, 2013 at the Leibniz-Institut DSMZ-Deutsche Sammlung von Mikroorganismen und Zellkulturen GmbH with the deposit number (cod. DSMZ 27925).

The method according to the present invention advantageously allows the production of fermented table olives, wherein in case of olives of the Greek Kalamata cultivar the yeast strain is *Saccharomyces cerevisiae* Kalamata-Y1 (KI-30-16) (cod. DSMZ 27801) and the strain of lactic acid bacterium is *Leuconostoc mesenteroides* (cod. DSMZ 27926).

The *Saccharomyces cerevisiae* yeast strain for fermentation of Greek olives of Kalamata cultivar was filed on October 22, 2013 at the Leibniz-Institut DSMZ-Deutsche Sammlung von Mikroorganismen und Zellkulturen GmbH with the deposit number *Saccharomyces cerevisiae* Kalamata-Y1 (KI-30 -16) (cod. DSMZ 27801).

The bacterial strain *Leuconostoc mesenteroides* for fermentation of Greek olives of Kalamata cultivar was filed on October 22, 2013 at the Leibniz-Institut DSMZ-Deutsche Sammlung von Mikroorganismen und Zellkulturen GmbH with the deposit number (cod. DSMZ 27926).

Use of the selected yeasts and bacteria and of the proposed production protocol allow: i) to obtain final products with increased and improved organoleptic and nutritional properties compared to a product obtained by natural fermentation; ii) to carry out the fermentation process in a shorter time than required to complete the process under natural conditions; iii) to carry out a more efficient and controllable fermentation process.

In the present description, the term "starter" or "starter culture" refers to a preparation containing live and viable microorganisms to be used with the objective of exploiting microbial metabolism to initiate fermentation and ensure the technological result of the production process.

In a preferred form of realization, in the method according to the present invention the concentration of yeasts, required in step b. for inoculation of the product obtained from step a, is between 5 x 10⁵ and 1 x 10⁶ Colony Forming Units (CFU) per milliliter (ml) of brine.

In a further preferred form of realization, in the method according to the present invention the concentration of lactic acid bacteria required in step d for inoculation. of the fermented product obtained from step c, is between 5 x 10⁵ and 1 x 10⁶ Colony Forming Units (CFU) per milliliter (ml) of brine.

The yeast strains used to carry out fermentation of olives of Leccino and Kalamata cultivars are prepared by growth in defined medium at pH 5 in presence of increasing salt concentrations from 0 up to 6% (weight/volume) NaCl, in order to adapt them to the conditions of salinity in which they are inoculated.

The lactic acid bacterial strains used to carry out fermentation of olives of Leccino and Kalamata cultivars are prepared by growth in defined medium in presence of increasing salt concentrations, from 0 up to 6% (weight/volume) NaCl, and decreasing pH, starting from pH 6 to pH 4-4.5, in order to adapt them to the conditions of salinity and acidity in which they are inoculated.

For both cultivars, fermentation is carried out at temperature ranging between 6 and 30°C, following seasonal temperature patterns.

The pilot scale fermentation, carried out for both Leccino and Kalamata olive cultivars, was inoculated with starters according to the procedure described in the present invention, using as control a pilot scale fermentation allowed to occur spontaneously.

Soluble sugars such as glucose, fructose and sucrose represent the substrate for microbial fermentation during which secondary metabolites are produced that are responsible for the organoleptic properties and the characteristic flavour of the drupe. As shown in Figures 4 and 5, glucose is the predominant sugar in the pulp of both olive cultivars, followed by fructose and sucrose. The total sugar content in the pulp of Leccino olives subjected to fermentation controlled by starters or to spontaneous fermentation varies from an initial content of 71.1 mg/g (dry weight), to 10.8 and 16.7 mg/g (dry weight), respectively (Fig. 4). Differently, in the case of the Kalamata cultivar, the initial total sugar content (65.5 mg/g dry weight) is metabolized completely after controlled or spontaneous fermentation process (Fig. 5). In the brine of Leccino cultivar, after starter-controlled or spontaneous fermentation, there is nearly a similar amount of fructose and undetectable sucrose, while the glucose content shows significant quantitative differences consisting in a total consumption at the end of starter-controlled fermentation and a residual glucose content of 0.69 g/L after spontaneous fermentation. The presence of a low sugar amount confirms the good performance of the fermentation process.

Depending on cultivar and maturity stage, organic acid content shows quantitative variations. The following organic acids have been identified in the pulp and in the brine of both cultivars: citric, tartaric, malic, succinic and acetic acid which decrease in the pulp during the fermentation process, while increasing in the brine. In particular, succinic acid is predominant in the brine of Leccino cultivar, while acetic acid is predominant in the Kalamata cultivar (Fig. 4 and 5). In the sample of Leccino cultivar, lactic acid was measured in pulp and brine after starter-controlled fermentation (12.2 mg/g dry weight in pulp and 2.8 mg/g dry weight in brine) compared to spontaneous fermentation (7.4 mg/g dry weight in pulp and 0.6 mg/g dry weight in brine). Instead, a higher concentration of lactic acid is present in the brine of Kalamata cultivar at the end of spontaneous fermentation (16.5 mg/g dry weight in pulp and 3.9 mg/g dry weight in brine) compared to controlled fermentation (16,0 mg/g dry weight in the pulp and 2.4 mg/g dry weight in the brine). Ethanol and glycerol have been identified in starter-controlled and spontaneous fermentation of brine of Leccino cultivar and both increase during the fermentation process (Fig. 4). In brine of Kalamata cultivar, the variation of ethanol concentration shows the same trend in both spontaneous and starter-controlled fermentation, while glycerol production was observed only in starter-controlled fermentation (Fig. 5).

For illustrative purpose, examples of embodiments of the present invention are provided below.

### EXAMPLES

### Example 1: Selection of yeasts and lactic acid bacteria to be used as starter for fermentation of table olives

Authors have developed an innovative selection protocol of yeasts and LAB to obtain microorganisms capable of surviving under conditions of low pH, high salt concentrations, and in presence of anti-microbial compounds such as mono- and polyphenols contained in olives and brine, and in presence of low nutrient content (sugars, organic acids, etc.) (Fig. 1).

Isolation of microorganisms on suitable media:
- First step: selection of microorganisms obtained on synthetic brines containing specific amounts of salt, of mono and polyphenolic species especially found in fermented brines, at acidic pH and low temperature. For selection of yeasts, the formulation of model brines consists of: 100 mg/L tyrosol, 30 mg/L caffeic acid, 1000 mg/L oleuropein, 500 mg/L verbascoside, 8-10% NaCl, 3 g/L glucose, 0.5 g/L yeast extract, 20 g/L agar, pH 4-4.5, growth at 14°C.
   For selection of lactic acid bacteria, the formulation of model brines consists of: 100 mg/L tyrosol, 15 mg/L caffeic acid, 300 mg/L oleuropein, 100 mg/L verbascoside, 8% NaCl, 3 g/L glucose, 0.5 g/L yeast extract, 20 g/L agar, pH 4.2, growth at 12°C
- Second step: selection of microorganisms by assessing the presence of positive characters such as i) presence of beta-glucosidase activity and consequent ability to degrade oleuropein (which is the primary reason for bitter taste), ii) presence of lipase activity, iii) absence of biogenic amine production, iv) absence of protease activity.
- Third step: identification of the selected microorganisms at the level of genus and species using molecular methods for DNA isolation, PCR amplification of rDNA ITS regions for yeasts and of 16S rDNA region for lactic acid bacteria, sequencing and sequence comparison with existing databases in order to identify GRAS (Generally Recognized As Safe) yeasts and bacteria.
- Fourth step. Use of yeasts and LAB thereby selected, identified and endowed with the best technological characteristics, to carry out fermentation tests on a pilot scale of 180 kg of olives.

For each olive cultivar taken into consideration, one indigenous yeast strain and one lactic bacterium strain were selected to be used in fermentation.

Suitable inocula of yeasts and LAB, approximately 10⁶ CFU/ml of brine, were prepared to perform these tests. During growth of the organisms, salinity values were increased and pH values were simultaneously decreased in order to ensure better adaptability of the selected starters to the microsystem brine/olive.

### Example 2: Fermentation protocol

The fermentation protocol that has been developed consists of the following operational phases:
1. Preparation of the sample of olives and brine
2. Inoculation with the yeast culture. The performance of the process is monitored every 7 days through the evaluation of: a) total count of yeasts, b) chemical and physical parameters such as temperature, pH and salinity. At the end of the first fermentation (alcoholic), the dominance of the inoculated yeast starter strain is evaluated in comparison with the total population.
3. Analysis of: a) variation of the concentration of sugars, organic acids, alcohols, mono- and polyphenols and isoprenoid compounds present in the brines and in the olives, by HPLC analysis, b) volatile and sensorial profile of olives and brines, by GC-MS analysis, c) presence of enterococci and other pathogenic bacteria.
4. Inoculation with the lactic acid bacterial culture follows at the end of the fermentation by yeast.
5. Monitoring of: a) total count of yeast and bacteria, b) physical-chemical parameters such as temperature, pH and salinity. At the end of the second fermentation (lactic), the dominance of the inoculated lactic bacterium starter strain is evaluated in comparison with the total population.
6. Analysis of: a) variation of the concentration of sugars, organic acids, alcohols, mono- and polyphenols and isoprenoid compounds present in brines and in the olives, by HPLC analysis, b) volatile and sensorial profile of olives and brines, by GC-MS analysis, c) presence of enterococci and other pathogenic bacteria.
7. Moreover, the correct performance of the process can be followed through the analysis of chemical descriptors associated with drupes and with the specific metabolism of yeasts and lactic acid bacteria (Fig. 8A, B):
   - Aldehydes: indicators of early stage of fermentation
   - Higher alcohols and terpenes: indicators of the first fermentation by yeast;
   - Free fatty acids, esters and alcohols: indicators of the second fermentation by lactic acid bacteria.

### Example 3: indigenous yeasts and lactic acid bacteria used

Indigenous yeasts and lactic acid bacteria used in this invention were deposited at DSMZ, Germany, at the Leibniz-Institut DSMZ-Deutsche Sammlung von Mikroorganismen und Zellkulturen GmbH, on October 22, 2013 and with the reference numbers below.

Starter combinations are:
*Saccharomyces cerevisiae* (cod. DSMZ 27800) and *Lactobacillus plantarum* (cod. DSMZ 27925) for fermentation of Italian olives of Leccino cultivar; *Saccharomyces cerevisiae* (cod. DSMZ 27801) and *Leuconostoc mesenteroides* (cod. DSMZ 27926) for fermentation of Greek olives of Kalamata cultivar. Use of these associations of microorganisms made possible to carry out effectively the first fermentation by yeast in a time of about 2 months and the second fermentation by lactic acid bacteria in 1 month. The resulting table olives showed better organoleptic and nutritional characteristics than those obtained by a natural fermentation process.

### Example 4

For the pilot scale fermentation of 180 kg of olives of the Italian Leccino cultivar, the *Saccharomyces cerevisiae* starter culture (cod. DSMZ 27800) was grown in YPD medium (1% yeast extract (weight/volume), 2% Peptone, 2% Glucose) at 28°C under shaking, using a salt concentration for adaptation of 6% of NaCl (weight/volume), and was inoculated at a concentration of 10⁶ CFU/ml in the sample of olives and brine. Within 60 days, the concentration of yeast cells remained almost constant and subsequently decreased during the last 30 days until about 10⁴ CFU/ml (Fig 2). Inoculation of the starter culture of *Lactobacillus plantarum* (Cod DSMZ 27925) was made at the 60th day of fermentation when GC-MS analysis detected the end of fermentation by yeast (Fig. 11). The LAB was grown in liquid MRS at 28°C and pH 4-4.5, at a salt concentration of 6% NaCl (weight/volume) and was inoculated at a concentration of 10⁶ CFU/ml in the sample of olives and brine. An increase of the population up to about 2 x 10⁷ CFU/ml was recorded over 30 days of lactic fermentation (Fig. 2).

At the end of alcoholic fermentation, the yeast population present in brine has been genetically characterized. For this, after brine was applied to an agar medium, 40 colonies were isolated, cultured in liquid medium and the genomic DNA was extracted. The different isolates were analyzed by RAPD-PCR using oligonucleotide (ATG)₅. The amplification products were analyzed by agarose gel electrophoresis, and a comparison with the profile of the inoculated control strain showed that the *Saccharomyces cerevisiae* (cod DSMZ 27800) strain dominated the fermentation process with 60% capacity compared to other yeasts present (Fig. 3 A). Likewise, the analysis of dominance performed as above [by use of oligonucletide (GTG)₅] revealed that the starter strain (*Lactobacillus plantarum* cod. DSMZ 27925) dominated fermentation with 60% capacity compared to other LAB present (Fig. 3 B).

For the pilot scale fermentation of 180 kg of Greek olives of Kalamata cultivar, the *Saccharomyces cerevisiae* starter culture *(cod.* DSMZ 27801) was grown in YPD medium (1% yeast extract (weight/volume), 2% Peptone, 2% Glucose) at 28°C under shaking, using a salt concentration for adaptation of 6% of NaCl (weight/volume), and was inoculated at a concentration of 10⁶ CFU/ml in the sample of olives and brine. Within 60 days, the concentration of the yeast remained almost constant, and then decreased in the last 30 days until about 8 x 10⁴ CFU/ml (Fig 2). Inoculation of the starter culture of *Leuconostoc mesenteroides* (cod DSMZ 27926) was made at the 60th day of fermentation, when GC-MS analysis detected the end of fermentation by the yeast (Fig. 12). The LAB was grown in liquid MRS at 28°C and pH 4-4.5, at a salt concentration of 6% NaCl (weight/volume) and was inoculated at a concentration of 10⁶ CFU/ml in the sample of olives and brine. An increase of the population up to about 1.3 x 10⁷ CFU/ml was recorded over 30 days of lactic fermentation (Fig. 2).

At the end of alcoholic fermentation, the yeast population present in brine has been genetically characterized. For this, after brine was applied to an agar medium, 40 colonies were isolated, cultured in liquid medium and the genomic DNA was extracted. The different isolates were analyzed by RAPD-PCR using oligonucleotide (ATG)₅. The amplification products were analyzed by agarose gel electrophoresis, and a comparison with the profile of the inoculated control strain showed that the *Saccharomyces cerevisiae* (cod. DSMZ 27801) strain dominated the fermentation process with 60% capacity compared to other yeasts present (Fig. 3 C). Likewise, the analysis of dominance performed as above [by use of oligonucletide (GTG)₅] revealed that starter strain (*Lactobacillus Leuconostoc mesenteroides* (cod. DSMZ 27926) dominated the fermentation process with 60% capacity compared to other LAB present (Fig. 3 D).

At the end of fermentations driven by starters, olives were completely debittered compared to two controls consisting of spontaneously fermented olives, indicating reduction of the time required for carrying out the debittering process to a maximum of three months.

### Example 5

The following polyphenolic compounds have been identified and quantified for both fermentations described in Example 4 of olives of Leccino cultivar respectively inoculated with starters or subjected to spontaneous fermentation: hydroxytyrosol, pyrocatechol, verbascoside, isoverbascoside, rutin, luteolin-7-O-glucoside and quercetin.

Quercetin, hydroxytyrosol and verbascoside are the compounds present in relevant concentrations, and in particular quercetin increases during both starter-controlled and spontaneous fermentation with a final content of 3,4 and 4.8 mg/g dry weight, respectively, at the end of the fermentation process. Hydroxytyrosol increases during the fermentation processes, with a final content of 1.6 mg/g dry weight at the end of starter-controlled fermentation and of 1.1 mg/g dry weight after spontaneous fermentation. Verbascoside has an almost constant content during the two fermentation processes (Fig. 6).

The following polyphenols were identified and quantified in the brine from Leccino cultivar: hydroxytyrosol, pyrocatechol, verbascoside, tyrosol, vanillic acid and caffeic acid. Hydroxytyrosol is the predominant compound with a content of 519.7 and 548.4 µg/mL at the end of starter-controlled and spontaneous fermentation, respectively. In the two fermentation processes all other phenolic compounds show the same trend except for pyrocatechol that was not identified in brine after spontaneous fermentation (Fig. 6).

The phenolic acids identified and quantified in the pulp of Kalamata cultivar, subjected to starter-controlled and spontaneous fermentation, were: hydroxytyrosol verbascoside, isoverbascoside, quercetin, tyrosol, with almost similar content in the two fermentations. Vanillic acid was detected only in the pulp subjected to starter-controlled fermentation, with a content of 0.4 mg/g dry weight. The compounds identified and quantified in brine of the Kalamata cultivar were hydroxytyrosol and tyrosol in similar amounts in the two fermentations, whereas pyrocatechol appeared after 60 days of spontaneous fermentation and remained constant at the end of fermentation (44,8 µg/mL). However, brine from Kalamata cultivar has a higher hydroxytyrosol content than brine from Leccino cultivar, consisting in 898.1 and 884.1 µg/mL, respectively, at the end of starter-controlled and spontaneous fermentation (Fig. 7).

### Example 6

Figure 8 shows the content in carotenoids (xanthophylls and carotenes) during starter-controlled and spontaneous fermentation in Leccino and Kalamata cultivars. Lutein, zeaxanthin and beta carotene carotenoids were identified. In particular 9-cis beta-carotene, a beta carotene degradation product, was detected only in Leccino cultivar after both starter-controlled and spontaneous fermentation. Approximately a three-fold higher total carotenoid content was found in Leccino cultivar compared to the Kalamata cultivar. Regarding olives of Leccino cultivar, lutein degradation is slower in starter-controlled than spontaneous fermentation, while beta carotene increases during starter-controlled fermentation from 2.2 to 4.8 g/g dry weight and remains almost constant in spontaneous fermentation. Finally zeaxanthin and of 9-cis beta-carotene contents are similar and remain constant in both fermentation processes (Fig. 8A, B). In the Kalamata cultivar, carotenoids show a similar trend except that zeaxanthin is degraded completely in spontaneous fermentation while it remains almost constant in starter-controlled fermentation (Fig. 8C, D).

As for the total vitamin E content at the end of the fermentation process, olives of Kalamata cultivar have a higher content than those of Leccino cultivar. The following isomeric forms of vitamin E were identified in both cultivars: beta and gamma tocotrienol, beta and gamma tocopherol, alpha tocopherol. Alpha-tocopherol is the predominant isomeric form in olives of Leccino cultivar, and shows the same degradation trend in both starter-controlled and spontaneous fermentation. At the end of the starter-controlled fermentation, beta and gamma tocotrienol show a lower level (19.5 µg/g dry weight) compared to spontaneous fermentation (25.3 µg/g dry weight); on the contrary, beta and gamma tocopherol show a higher level (13.1 µg/g dry weight) at the end of starter-controlled fermentation compared to spontaneous fermentation (9.7 µg dry weight) (Fig. 9A, B). In olives of Kalamata cultivar, alpha-tocopherol shows an almost constant profile during both fermentation processes. Beta and gamma tocotrienol decrease during starter-controlled and spontaneous fermentation, reaching values of 79.9 and 57.6 µg/g dry weight, respectively, at the end of fermentation. Finally, beta and gamma tocopherol increase during the fermentation process and, at the end of the process, show higher concentrations in starter-controlled fermentation (28.2 µg dry weight) compared to spontaneous fermentation (15.3 µg dry weight) (Fig. 9 C, D).

Based on above data, it is possible to conclude that, in both olive cultivars tested, starter-controlled fermentation allows to maintain or increase bioactive molecules belonging to carotenoid and vitamin E classes.

### Example 7

The extraction, identification and quantification of volatile molecules, which represent secondary fermentation products, were done by the SPME (solid phase micro-extraction)-GC-MS system, consisting of a 65 µm PDMS-DVB SPME fiber, a gas chromatograph (GC) equipped with a HP INNOWax polar column and a quadrupole mass spectrometer (MS). Peak identification was performed by comparing mass spectra with the spectra in the NIST98 database and with those of pure standard molecules.

During fermentation, the following classes of compounds were identified by GC-MS in the pulp of Leccino and Kalamata olives: aldehydes, ketones, alcohols, esters, fatty acids, hydrocarbons, volatile phenols and terpenes. In the course of starter-inoculated fermentation of olives of Leccino cultivar, an increase in concentration of volatile compounds belonging to classes of alcohols, esters, fatty acids and hydrocarbons was observed to be significantly higher compared to spontaneous fermentation (Fig 11A, B). In particular, the concentrations in starter-driven fermentation and spontaneous fermentation were respectively:
- aldehydes were approximately 27 µg/Kg in starter-inoculated fermentation and 30 µg/Kg in natural fermentation;
- Alcohols were approximately 300 µg/Kg in starter driven fermentation and 250 µg/Kg in natural fermentation;
- Esters were present at concentrations above 200 µg/Kg in starter driven fermentation and above 100 µg/Kg in natural fermentation;
- Fatty acids were present in significant amounts only in the starter inoculated fermentations.

Differences in concentration of such molecules influenced the sensorial quality of the finished product, generating two significantly different sensorial profiles. In particular, the profile associated with olives produced by starters is much richer in fruity odor (associated with esters), herbaceous odor, sweet, winey (associated with the class of alcohols), with notes of fat associated with fatty acids and herbaceous generated by aldehydes (Fig. 11C, D).

An increase in concentration of volatile compounds belonging to the classes of alcohols, esters, fatty acids, hydrocarbons and volatile phenols has been observed during starter-inoculated fermentation of olives of Kalamata cultivar and was significantly higher than in spontaneous fermentation (Fig 12A, B). In particular, gas-chromatographic analyses carried out on both starter-driven and spontaneous fermentation showed the following for each class of compounds analyzed:
- aldehydes at the end of fermentation were about 27.50 µg/Kg in starter inoculated fermentation and 21.72 µg/Kg in spontaneous fermentation;
- Alcohols were approximately 327.32 µg/Kg in starter inoculated fermentation and 228.14 µg/Kg in spontaneous fermentation;
- Esters were found in a concentration of 277.95 µg/Kg in starter inoculated fermentation and 125.13 µg/Kg in spontaneous fermentation;
- fatty acids were produced in concentrations of about 39 µg/Kg in starter driven fermentation and 22 µg/Kg in spontaneous fermentation;
- Terpenes, varietal compounds present in both free and combined forms, were found in the amount of 61.45 µg/Kg in starter inoculated fermentation and 46 µg/Kg in the spontaneously fermented control;
- Hydrocarbons were found in the amount of approximately 51 µg/Kg in starter-driven fermentation and 20 µg/Kg in the sample fermented in spontaneous mode.

Differences in concentration of such molecules influenced the sensorial quality of the finished product, generating two significantly different sensorial profiles. In particular the profile associated with the olives produced by starters is much richer in fruity odor (associated with esters), herbaceous, sweet, winey odors (associated with the class of alcohols), with notes of fat associated with fatty acids and herbaceous generated by the aldehydes, and a floral note generated by terpenes (Fig. 12C, D).

The results derived from the above examples show the clear advantages of the method described according to the present invention over current empirical practice for production of table olives. Said method has proven surprisingly and advantageously suited for fermentation of table olives, it has proven to be fast and extremely simple to perform, and it can be conveniently applied in any type of production plant.

## Claims

1. Method for producing fermented table olives comprising the steps of:
a. soaking the olives in brine;
b. inoculating the product obtained in step a. with a yeast culture;
c. incubating the product obtained in step b. in order to perform the alcoholic fermentation
d. inoculating the fermented product obtained in step c. using a Lactic Acid Bacterium (LAB) culture;
e. incubating the product obtained in step d. to in order to perform the lactic fermentation.

2. The method for producing fermented table olives according to claim 1 comprising the steps of:
a. soaking the olives in brine wherein the olive: brine ratio of step a. ranges from 3:1 using olives with a higher caliber to 2:1 for olives with a smaller caliber;
b. inoculating the product deriving from step a. with a yeast culture, wherein the volume ratio between yeast culture volume/brine in step b. corresponds to 1/300;
c. incubating the product obtained in step b. at a temperature that can range from 6°C to 30°C and for a time period that can vary from 45 to 60 days in order to carry out the alcoholic fermentation;
d. inoculating the fermented product obtained in step c. with a lactic bacterium using a starter LAB culture/fermented product ratio of 1/300;
e. incubating the product obtained in step d. to perform lactic fermentation at a temperature that can range from 6°C to 30°C and for a time period that can vary from 15 to 30 days.

3. The method according to anyone of claims 1 or 2, wherein the olives used are chosen from the group consisting of the Italian cultivar Leccino and the Greek cultivar Kalamàta.

4. The method according to anyone of claims 1, 2 or 3, wherein when the olives used belong to the Italian cultivar Leccino, the yeast culture is the strain *Saccharomyces cerevisiae* (cod. DSMZ 27800) and the LAB culture is the strain *Lactobacillus plantarum* (cod. DSMZ 27925).

5. The method according to anyone of claims 1, 2 or 3, wherein when the olives used belong to the Greek cultivar Kalamata, the yeast culture is the strain *Saccharomyces cerevisiae* (cod. DSMZ 27801) and the LAB culture is the strain *Leuconostoc mesenteroides* (cod. DSMZ 27926).

6. The method according to anyone of claims 1 to 5, wherein the yeast strain concentration suitable to perform step b. of inoculating the product obtained in the step a., ranges from 5 x 10⁵ to 1 x 10⁶ Colony Forming Units (CFU) per milliliter of brine.

7. The method according to anyone of claims 1 to 6, wherein the lactic bacteria concentration suitable to perform step d. of inoculating the fermented product obtained in step c., ranges from 5 x 10⁵ to 1 x 10⁶ Colony Forming Units (CFU) per milliliter of brine.

## Patentansprüche

1. Verfahren zur Herstellung fermentierter Tafeloliven, folgende Schritte aufweisend:
a. Einweichen der Oliven in einer Lake;
b. Inokulation des in Schritt a. erhaltenen Produkts mit einer Hefekultur;
c. Inkubieren des in Schritt b. erhaltenen Produkts zum Durchführen einer alkoholischen Fermentation;
d. Inokulation des in Schritt c. erhaltenen, fermentierten Produkts unter Verwendung einer Milchsäurebakterien (LAB) - Kultur;
e. Inkubieren des in Schritt d. erhaltenen Produkts zum Durchführen einer Milchsäure - Fermentation.

2. Verfahren zur Herstellung fermentierter Tafeloliven gemäß Anspruch 1, folgende Schritte aufweisend:
a. Einweichen der Oliven in einer Lake, wobei das Verhältnis von Oliven: Lake von 3 : 1 bei der Verwendung von größeren Oliven bis 2 : 1 bei kleineren Oliven reicht;
b. Inokulation des in Schritt a. entstandenen Produkts mit einer Hefekultur, wobei das Volumenverhältnis zwischen Hefekulturvolumen / Lake in Schritt b. 1 / 300 entspricht;
c. Inkubieren des in Schritt b. erhaltenen Produkts bei einer Temperatur zwischen 6°C und 30°C über einen Zeitraum von 45 bis 60 Tagen, zum Durchführen einer alkoholischen Fermentation;
d. Inokulation des in Schritt c. erhaltenen, fermentierten Produkts mit einem Milchsäurebakterium unter Verwendung eines Verhältnisses von LAB - Kultur / fermentiertem Produkt von 1 / 300;
e. Inkubieren des in Schritt d. erhaltenen Produkts zum Durchführen der Milchsäure - Fermentation bei einer Temperatur zwischen 6°C und 30°C über einen Zeitraum von 15 bis 30 Tagen.

3. Verfahren gemäß einem der Ansprüche 1 oder 2, wobei die verwendeten Oliven ausgewählt werden aus einer Gruppe bestehend aus der italienischen Sorte Leccino und der griechischen Sorte Kalamàta.

4. Verfahren gemäß einem der Ansprüche 1, 2 oder 3, wobei die verwendeten Oliven zur italienischen Sorte Leccino gehören, der Stamm der Hefekultur *Saccharomyces cerevisiae* (cod. DSMZ 27800) ist und der Stamm der LAB-Kultur *Lactobacillus plantarum* (cod. DSMZ 27925) ist.

5. Verfahren gemäß einem der Ansprüche 1, 2 oder 3, wobei die verwendeten Oliven zur griechischen Sorte Kalamàta gehören, der Stamm der Hefekultur *Saccharomyces cerevisiae* (cod. DSMZ 27801) ist und der Stamm der LAB-Kultur *Leuconostoc mesenteroides* (cod. DSMZ 27926) ist.

6. Verfahren gemäß einem der Ansprüche 1 bis 5, wobei die Konzentration des Hefestamms, die geeignet ist, im Schritt b. das Inokulieren des in Schritt a. entstandenen Produkts durchzuführen, von 5 x 10⁵ bis 1 x 10⁶ koloniebildende Einheiten (CFU) pro Milliliter Lake reicht.

7. Verfahren gemäß einem der Ansprüche 1 bis 6, wobei die Konzentration der Milchsäurebakterien, die geeignet ist, im Schritt d. das Inokulieren des in Schritt c. erhaltenen, fermentierten Produkts durchzuführen, von 5 x 10⁵ bis 1 x 10⁶ koloniebildende Einheiten (CFU) pro Milliliter Lake reicht.

## Revendications

1. Procédé de production d'olives de table fermentées comprenant les étapes consistant à :
a. tremper les olives dans de la saumure ;
b. inoculer le produit obtenu à l'étape a. avec une culture de levure ;
c. incuber le produit obtenu à l'étape b. afin d'effectuer la fermentation alcoolique ;
d. inoculer le produit fermenté obtenu à l'étape c. en utilisant une culture bactérienne d'acide lactique (LAB) ;
e. incuber le produit obtenu à l'étape d. pour effectuer la fermentation lactique.

2. Procédé de production d'olives de table fermentées selon la revendication 1, comprenant les étapes consistant à :
a. tremper les olives dans de la saumure, dans lequel le rapport olive : saumure de l'étape a. est dans la plage de 3:1 en utilisant des olives avec un calibre supérieur à 2:1 pour des olives avec un calibre plus petit ;
b. inoculer le produit issu de l'étape a. avec une culture de levure, dans lequel le rapport en volume entre le volume de culture de levure/saumure à l'étape b. correspond à 1/300 ;
c. incuber le produit obtenu à l'étape b. à une température qui peut aller de 6 C à 30 C, et pendant une période qui peut varier de 45 à 60 jours, afin de réaliser la fermentation alcoolique ;
d. inoculer le produit fermenté obtenu à l'étape c. avec une bactérie lactique en utilisant un rapport culture LAB de démarrage/ produit fermenté de 1/300 ;
e. incuber le produit obtenu à l'étape d. pour effectuer une fermentation lactique à une température qui peut être dans la plage de 6 C à 30 C, et pendant une période de temps qui peut varier de 15 à 30 jours.

3. Procédé selon l'une quelconque des revendications 1 ou 2, dans lequel les olives utilisées sont choisies dans le groupe constitué par le cultivar italien Leccino et le cultivar grecque Kalamàta.

4. Procédé selon l'une quelconque des revendications 1, 2 ou 3, dans lequel, lorsque les olives utilisées appartiennent au cultivar italien Leccino, la culture de levure est la souche *Saccharomyces cerevisiae* (cod. DSMZ 27800) et la culture LAB est la souche *Lactobacillus plantarum* (cod. DSMZ 27925).

5. Procédé selon l'une quelconque des revendications 1, 2 ou 3, dans lequel, lorsque les olives utilisées appartiennent au cultivar grecque Kalamàta, la culture de levure est la souche *Saccharomyces cerevisiae* (cod. DSMZ 27801) et la culture LAB est la souche *Leuconostoc mesenteroides* (cod DSMZ 27926).

6. Procédé selon l'une quelconque des revendications 1 à 5, dans lequel la concentration de souche de levure appropriée pour effectuer l'étape b. consistant à inoculer le produit obtenu dans l'étape a., est comprise entre 5 x 10⁵ et 1 x 10⁶ unités formatrices de colonies (CFU) par ml de saumure.

7. Procédé selon l'une quelconque des revendications 1 à 6, dans lequel la concentration de bactéries lactiques appropriées pour effectuer l'étape d. consistant à inoculer le produit fermenté obtenu dans l'étape c est comprise entre 5 x 10⁵ et 1 x 10⁶ unités formatrices de colonies (CFU) par ml de saumure
